# EUROPEAN PATENT SPECIFICATION

(11) **EP 2 810 917 B1**
(45) Date of publication and mention of the grant of the patent: **13.04.2016**
(21) Application number: 14162300.9
(22) Date of filing: 28.03.2014
(51) Int. Cl.: C01B 25/32, A61L 27/02, A61Q 11/00, A61F 2/28

(54) **A method for producing calcium hydroxyapatite nano-crystals of the desired morphology**
Verfahren zur Herstellung von Kalcium-Hydroxyapatit-Nanokristallen in gewünschter Morphologie
Procédé d'obtention des nanocristaux d'hydroxyapatite de calcium de morphologie désirée

(30) Priority: 04.06.2013 LT 2013060
(43) Date of publication of application: 10.12.2014
(73) Proprietor: UAB Moksliniu Tyrimu Pletros Laboratorija, 01128 Vilnius (LT)
(72) Inventor: KAREIVA, Aivaras, 05255 Vilnius (LT)
(74) Representative: Lenkutiene, Marija

(56) References cited:
- I. BOGDANOVI6IENE; A. BEGANSKIENE; K. T6NSUAADU; J. GLASER; H.-J. MEYER; A. KAREIVA: "Calcium hydroxyapatite, Ca10(PO4)6(OH)2 ceramics prepared by aqueous sol-gel processing", MATER. RES. BULL., vol. 41, 9 March 2006 (2006-03-09), page 1754, XP0028077930,
- IRMA BOGDANOVICIENE ET AL: "pH impact on the sol-gel preparation of calcium hydroxyapatite, Ca10(PO4)6(OH)2, using a novel complexing agent, DCTA", CENTRAL EUROPEAN JOURNAL OF CHEMISTRY, vol. 8, no. 6, 1 December 2010 (2010-12-01), pages 1323-1330, XP055143681, ISSN: 1895-1066, DOI: 10.2478/s11532-010-0113-0
- Irma Bogdanoviciene ET AL: "Influence of heating conditions on the formation of sol- gel derived calcium hydroxyapatite", , 1 January 2010 (2010-01-01), pages 98-105, XP055143687, Retrieved from the Internet: URL:http://www.lmaleidykla.lt/publ/0235-72 16/2010/2-4/98-105.pdf [retrieved on 2014-10-01]

## Description

### Tecnical Field

The invention relates to chemical compounds characterised by a porous structure, specifically, to producing calcium hydroxyapatite nano-crystals of the desired morphology intended for the treatment of skeletal disorders.

By chemical composition, calcium-containing apatites are close to the composition of the non-organic part of bone tissue and show similar chemical and physical properties; therefore, their use in the production of skeletal implants and in odontology is very promising.

### Background Art

The most popular method for the synthesis of calcium hydroxyapatite crystals is the synthesis of calcium hydroxyapatite sol-gels in a non-aqueous medium, while using various dialkyl phosphites as initial phosphorous substances. Ca-P-O gels for the synthesis of hydroxyapatite were obtained by slowly hydrolising mixtures of calcium butanoate or calcium acetylacetonate with dialkyl phosphite. Single-phase Ca₁₀(PO₄)₆(OH)₂ samples were obtained by heating the initial Ca-P-O gels for 15 hours at a temperature of 1000 °C, which is quite a low temperature to obtain compounds of this type (A. Beganskiene, I. Bogdanovičienè, S. Mathur, H. Shen, A. Kareiva. Dialkyl phosphates as phosphorous precursors for the sol-gel synthesis of calcium hydroxyapatite. Environmental Chemistry and Physics. 26 (2004) 164; A. Beganskiene, I. Bogdanovičienè, A. Kareiva. Calcium acetylacetonate - A novel calcium precursor for sol-gel preparation of Ca10(Po4)6(OH)2. Chemistry. 2-3 (2006) 16.). However, calcium hydroxyapatite crystals of the desired morphology were not produced by these methods.

Another method for producing calcium hydroxyapatite is known, where Ca(No₃)_{2·}0,4H₂O, (NH₄)₂HPO₄, NH₄OH, and methyl cellulose. In the course of the synthesis, 3 ml of 0.1 g/l of methyl cellulose solution was dissolved in 1440 ml of deionised water. In this solution, 0.152 mol Ca(No₃)₂·0,4H₂O was dissolved and 0.090 mol (NH₄)₂HPO₄ was added. The solution was continuously mixed on a hot heating stove. The obtained mixture was mixed additionally for another 3 hours at a temperature of 60-70 °C. The obtained residue was filtered and decanted with deionised water for 5 times. The filtrate was dried for 24 hours at a temperature of 100 °C. The obtained powder was heated for 6 hours at a temperature of 1000 °C with interim mixings. (C. C. Silva, A. S. B. Sombra. Raman spectroscopy measurements of hydroxyapatite obtained by mechanical alloying. J. Phys. Chem. Solids. 65 (2004) 1031.)

The obtaining of calcium hydroxyapatite by the sol-gels method is closest to the proposed invention. For the synthesis of Ca-P-O gels by the aqueous method, calcium acetate monohydrate and diammonium hydrogen tetroxo-phosphate were selected as the sources of Ca and P. The stoichiometric quantity of the initial substances Ca(CH₃CO₂)₂·H₂O (0.03 mol) and (NH₄)₂HPO₄ (0.018 mol) were separately dissolved in 100 ml of distilled water. The obtained transparent colourless solutions were mixed at the room temperature or when heated to 65 °C. Then 0.015 mol of tartaric acid was dissolved in 100 ml of distilled water and, after intensive stirring at the same temperature in covered glasses for one hour, the tartaric acid solution was poured into the solution of the initial substances. Then the solution was concentrated in an open glass at a temperature of 65 °C. The obtained Ca-P-O gels were dried in an oven (100 °C) for 24 hours. The xerogels were ground in an agate mortar and heated at a temperature of 1000 °C for 5 hours (the temperature increase speed is 10 °C/min). (I. Bogdanovičiene, A. Beganskiene, K. Tonsuaadu, J. Glaser, H.-J. Meyer, A. Kareiva. Calcium hydroxyapatite, (Ca10(PO4)6(OH)2, HA) ceramics prepared by aqueous sol-gel processing. Mater. Res. Bull. 41 (2006) 1754).

Nevertheless, calcium hydroxyapatite crystals of the desired morphology were not obtained by this method either.

### Summary of invention

The purpose of the proposed invention is to obtain calcium hydroxyapatite nano-crystals of the desired morphology and size.

The purpose may be achieved by preparing two solutions in the proposed method:
a) 2.5-5.0wt% of calcium acetate and 1.2-2.4wt% of diammonium hydrogen tetroxo-phosphate is separately dissolved in 80-120 ml of distilled water and stir them at a temperature of 25-65 °C; b) 1.15-2.25wt% of tartaric acid (TA) is dissolved in 90-100 ml of distilled water, and 4.4-8.8wt% of ethylenediaminetetraacetic acid (EDTA), 5.85-11.7wt% of 1,2- diaminocyclohexane-tetra-acetate (DCTA) is dissolved in 100 ml of water and 22 ml of 10% ammonia water solution, and stirred, pouring the solution b) into solution a), stirring for 15-20 hours at 65-75 °C, drying the obtained gel for 20-24 hours at 100 °C and, after it is grounded additionally heating for 5 hours at 900-1200 °C; when the molar ratio of DCTA, EDTA, and TA is 1:1:1, the size of the obtained crystals is 200-300 nm and the crystals have the shape of coalesced spheres; when the molar ratio of DCTA, EDTA, and TA is 1:0.5:0.5, the size of the obtained crystals is 200-400 nm and the crystals have the shape of planes; when the molar ratio of DCTA, EDTA, and TA is 1:0.25:0.005, the size of the obtained crystals is 20-80 nm and the crystals have the shape of spheres; when the molar ratio of DCTA, EDTA, and TA is 0.5:0.25:1, the size of the obtained crystals is 800-1000 nm and the the crystals have the shape of rods; when the molar ratio of DCTA, EDTA, and TA is 0.005:0.005:1, the size of the obtained crystals is 1-3 µm and the crystals have the shape of rods; when the molar ratio of DCTA, EDTA, and TA is 0.25:1:0.5, the size of the obtained crystals is 600-700 nm and the crystals have the shape of needles.

The synthesis of calcium hydroxyapatite by the proposed sol-gel method, it is possible to control the size of crystals from 20 nm to 3 µm; it also possible to control the morphology of particles and the shape of particles (plane, needle-like, spherical, and other crystals). It is achieved by effectively controlling the nature, concentrations, and ratio of complex-forming reagents.

Calcium hydroxyapatite is obtained by the aqueous sol-gel method, while selecting calcium acetate monohydrate and diammonium hydrogen tetroxo-phosphate as the sources of Ca and P. In the sol-gel synthesis processes, different complexing reagents were used: TA (tartaric acid), EDTA (ethylenediaminetetraacetic acid), and DCTA (1,2-diaminocyclohexane-tetra-acetate).

### Examples of embodiment

EXAMPLE: For the obtaining of calcium hydroxyapatite, two solutions were prepared: a) 0.03 mol Ca(CH₃CO₂)₂·H₂O' and 0.018 mol of (NH₄)₂HPO₄ were separately dissolved in 100 ml of distilled water; b) 0.015 mol of TA was dissolved in 100 ml of distilled water, and 0.03 mol of EDTA and 0.03 mol of DCTA was dissolved in 100 ml of water and 22 ml of 10% ammonia water solution. Transparent colourless solutions were obtained, which were stirred at the room temperature and then when heated up to 65 °C. The mixture discoloured to whitish. Two solutions of the initial substances were so prepared. After intensive stirring at the same temperature in covered glasses for one hour, TA, EDTA and DCTA solutions were poured into the solutions of the initial substances. Both solutions became more transparent. The obtained solutions were then stirred in a magnetic stirrer in covered laboratory glasses for 15 hours at a temperature of 65 °C. Then the solutions were concentrated in opened glasses at the same temperature of 65 °C. Stirring was finished when the solutions turned into white viscous gel. The obtained gels were dried in an oven (100 °C) for 24 hours. The obtained xerogels were ground in an agate mortar and additionally heated at a temperature of 1000 °C for 5 hours (the temperature increase speed is 10 °C/min).

By changing the molar ratios of the complex-forming reagents (DCTA, EDTA, and TA), it is possible to change the surface morphology of calcium hydroxyapatite (Table 1).

**Table 1**

| Item No. | Molar ratio of DCTA, EDTA, and TA | Shape of crystals | Size of crystals |
|---|---|---|---|
| 1. | DCTA:EDTA:TA=1:1:1 | Coalesced spheres | 200-300 nm |
| 2. | DCTA:EDTA:TA=1:0.5:0.5 | Planes | 200-400 nm |
| 3. | DCTA:EDTA:TA=1:0.25:0.005 | Spheres | 20-80 nm |
| 4. | DCTA:EDTA:TA=0.5:0.25:1 | Rods | 800-1000 nm |
| 5. | DCTA:EDTA:TA=0.005:0.005:1 | Rods | 1-3 µm |
| 6. | DCTA:EDTA:TA=0.25:1:0.5 | Needle-like | 600-700 nm |

The proposed method allows creating nano-structured calcium hydroxyapatite and effectively using it in medicine. 3D (three-dimensional) open ceramic holders for tissue cells were made of nano-CAHA. The formed 3D synthetic bone block is characterised by porous microstructure. Total porosity was approximately equal to 92.4 ± 2.2.%, and the average size of pores was 384 ± 74 µm. CAHA samples of different morphology can be used for the restoration of differently damaged bone tissue, whose structure depends on the age, gender, and other factors. Such blocks can be used in orthopaedic surgery as bone implants. The nano-CAHA synthesised by the sol-gel method can be used for the remineralisation of dental enamel. It was determined that the remineralisation of the CAHA produced by the proposed method equals to 31.3% ± 13.9%. CAHA samples synthesised by the sol-gel method and replaced with ions of other metals (Ca₁₀₋ₓMₓ(PO₄)₆(OH)₂ should be suitable for the transport of drugs and proteins in vivo.

## Claims

1. A method producing calcium hydroxyapatite nano-crystals of the desired morphology, comprising dissolving calcium acetate monohydrate and diammonium hydrogen tetroxo-phosphate in distilled water, stirring the obtained solution at 25-65 °C with addition of tartaric acid, concentrating the solution, drying the obtained gel, and its grinding at high temperature, **characterised in** preparing of two solutions:
a) 2.5-5.0wt% of calcium acetate and 1.2-2.4wt% of diammonium hydrogen tetroxo-phosphate is separately dissolved in 80-120 ml of distilled water and stirred at a temperature of 25-65 °C;
b) 1.15-2.25wt% of tartaric acid (TA) is dissolved in 90-100 ml of distilled water, and 4.4-8.8wt% of ethylenediaminetetraacetic acid (EDTA), 5.85-11.7wt% of 1,2-diaminocyclohexane-tetra-acetate (DCTA) is dissolved in 100 ml of water and 22 ml of 10% ammonia water solution, and stirred; pouring the solution b) into solution a), stirring for 15-20 hours at 65-75 °C, drying the obtained gel for 20-24 hours at 100 °C, and, after it is grounded additionally heating for 5 hours at 900-1200 °C.

2. The method according to Claim 1, **characterised in that** when the molar ratio of DCTA, EDTA, and TA is 1:1:1, the size of the obtained crystals is 200-300 nm and the crystals have the shape of coalesced spheres.

3. The method according to Claim 1 , **characterised in that** when the molar ratio of DCTA, EDTA, and TA is 1:0.5:0.5, the size of the obtained crystals is 200-400 nm and the crystals have the shape of planes.

4. The method according to Claim 1, **characterised in that** when the molar ratio of DCTA, EDTA, and TA is 1:0.25:0.005, the size of the obtained crystals is 20-80 nm and the crystals have the shape of spheres.

5. The method according to Claim 1, **characterised in that** when the molar ratio of DCTA, EDTA, and TA is 0.5:0.25:1, the size of the obtained crystals is 800-1000 nm and the crystals have the shape of rods.

6. The method according to Claim 1, **characterised in that** when the molar ratio of DCTA, EDTA, and TA is 0.005:0.005:1, the size of the obtained crystals is 1-3 µm and the crystals have the shape of rods.

7. The method according to Claim 1, **characterised in that** when the molar ratio of DCTA, EDTA, and TA is 0.25:1:0.5, the size of the obtained crystals is 600-700 nm and the crystals have the shape of needles.

## Patentansprüche

1. Verfahren zur Herstellung von Calciumhydroxyapatit-Nanokristallen der gewünschten Form, bei dem Calciumacetatmonohydrat und Diammonium-Hyrogentetroxophpsphat in destilliertem Wasser gelöst werden, die erhaltene Lösung bei 25-65 °C unter Zufügung von Weinsäure gerührt, konzentriert und das so erhaltene Gel getrocknet und dieses wiederum bei hoher Temperatur gemahlen werden, ist **dadurch gekennzeichnet, dass** zwei Lösungen bereitet werden:
a) 2,5 - 5,0 Gew% Calciumacetat und 1,2 - 2,4 Gew% Diammoniumhydrogentetroxophosphat werden jeweils einzeln in 80 - 120 ml destilliertem Wasser gelöst und bei einer Temperatur von 25 - 65 °C gerührt;
b) 1,15 - 2,25 Gew% Weinsäure (TA) werden in 90 - 100 ml destilliertem Wasser gelöst, und 4,4 - 8,8 Gew% Ethylenediaminetetraacetat (EDTA), 5,85 - 11,7 Gew% 1,2-Diaminocyclohexane-Tetraacetat (DCTA) in einer Lösung aus 100 ml Wasser und 22 ml 10-%iger Ammoniak-Wasser-Lösung und dann gerührt; Lösung b) wird daraufhin in Lösung a) geschüttet und 15 - 20 Stunden bei 65-75 °C gemixt, das erhaltene Gel 20 - 4 Stunden bei 100 °C getrocknet und nach dem grounded zusätzlich fünf Stunden lang bei 900 -1200 °C erhitzt.

2. Verfahren nach Anspruch 1, **dadurch gekennzeichnet, dass** dann, wenn das Molverhältnis zwischen DCTA, EDTA und TA 1:1:1 beträgt, die erhaltenen Kristalle 200 - 300 mm groß sind und die Form von verschmolzenen Kugeln haben;

3. Verfahren nach Anspruch 1, **dadurch gekennzeichnet, dass** dann, wenn das Molverhältnis zwischen DCTA, EDTA und TA 1:0,5:0,5 beträgt, die erhaltenen Kristalle 200 - 400 mm groß sind und die Form von Flächen haben;

4. Verfahren nach Anspruch 1, **dadurch gekennzeichnet, dass** dann, wenn das Molverhältnis zwischen DCTA, EDTA und TA 1:0,25:0,0005 beträgt, die erhaltenen Kristalle 20 - 80 mm groß sind und die Form von Kugeln haben;

5. Verfahren nach Anspruch 1, **dadurch gekennzeichnet, dass** dann, wenn das Molverhältnis zwischen DCTA, EDTA und TA 0,5:0,25:1 beträgt, die erhaltenen Kristalle 800 - 1000 mm groß sind und die Form von Stäben haben;

6. Verfahren nach Anspruch 1, **dadurch gekennzeichnet, dass** dann, wenn das Molverhältnis zwischen DCTA, EDTA und TA 0,005:0,005:1 beträgt, die erhaltenen Kristalle 1 -3 µm groß sind und die Form von Stäben haben;

7. Verfahren nach Anspruch 1, **dadurch gekennzeichnet, dass** dann, wenn das Molverhältnis zwischen DCTA, EDTA und TA 0,25:1:0,5 beträgt, die erhaltenen Kristalle 600 - 700 nm groß sind und die Form von Nadeln haben;

## Revendications

1. Le procédé d'obtention des nanocristaux d'hydroxyapatite de calcium de la morphologie désirée, comprenant la dissolution de l'acétate de calcium monohydraté et de diammonium hydrogène tetroxo-phosphate dans de l'eau distillée, en agitant la solution obtenue à des températures de 25 à 65 °C avec l'acide tartrique, en concentrant la solution , en séchant le gel obtenu, et en le broyant à haute température, est **caractérisé par** la préparation de deux solutions:
a) 2.5-5.0 % en poids d'acétate de calcium et 1.2-2.4 % en poids de diammonium hydrogène tetroxo-phosphate sont dissous séparément dans 80-120 ml d'eau distillée et en l'agitant à des températures de 25 à 65 °C;
b) 1.15-2.25 % en poids d'acide tartrique (AT) est dissous dans 90 à 100 ml d'eau distillée et 4.4-8.8 % en poids d'acide éthylène diamine tétraacétique (EDTA), 5.85-11.7 % en poids de 1,2 diaminocyclohexane tétra-acétate (DCTA) est dissous dans 100 ml d'eau et 22 ml de 10%
de solution aqueuse de l'ammoniac, et on l'agite;
en versant la solution b) dans la solution a), en agitant pendent 15-20 heures à des températures de 65 à 75 °C, en séchant du gel obtenu pendant 20-24 heures à une température de 100 °C, et, après l'avoir été broyé il été réchauffé pendant 5 heures à des températures de 900 à 1200 °C.

2. Procédé selon la revendication 1, **caractérisé en ce que** lorsque le rapport molaire du DCTA, l'EDTA, et AT est de 1: 1: 1, la taille des cristaux obtenus est de 200 à 300 nm et les cristaux ont la forme de sphères coalisées.

3. Procédé selon la revendication 1, **caractérisé en ce que** lorsque le rapport molaire du DCTA, l'EDTA, et AT est de 1: 0,5: 0,5, la taille des cristaux obtenus est de 200 à 400 nm et les cristaux ont la forme de plans.

4. Procédé selon la revendication 1, **caractérisé en ce que** lorsque le rapport molaire du DCTA, l'EDTA, et AT est de 1: 0,25: 0,005, la taille des cristaux obtenus est de 20 à 80 nm et les cristaux ont la forme de sphères.

5. Procédé selon la revendication 1, **caractérisé en ce que** lorsque le rapport molaire du DCTA, l'EDTA, et AT est de 0,5: 0,25: 1, la taille des cristaux obtenus est de 800 à 1000 nm et les cristaux ont la forme de tiges.

6. Procédé selon la revendication 1, **caractérisé en ce que** lorsque le rapport molaire du DCTA, l'EDTA, et AT est de 0,005: 0,005: 1, la taille des cristaux obtenus est de 1 à 3 µm et les cristaux ont la forme de tiges.

7. Procédé selon la revendication 1, **caractérisé en ce que** lorsque le rapport molaire du DCTA, l'EDTA, et AT est de 0,25: 1: 0,5, la taille des cristaux obtenus est de 600 à 700 nm et les cristaux ont la forme d'aiguilles.
